# EUROPEAN PATENT SPECIFICATION

(11) **EP 1 890 602 B1**
(45) Date of publication and mention of the grant of the patent: **12.10.2011**
(21) Application number: 06727901.8
(22) Date of filing: 12.04.2006
(51) Int. Cl.: A61B 5/11, A61B 5/024, A61B 5/08

(54) **PATIENT MONITORING SYSTEM**
PATIENTENÜBERWACHUNGSSYSTEM
SYSTEME DE SURVEILLANCE DE PATIENT

(30) Priority: 20.04.2005 EP 05103175
(43) Date of publication of application: 27.02.2008
(73) Proprietor: Philips Intellectual Property & Standards GmbH, 20099 Hamburg (DE); Koninklijke Philips Electronics N.V., 5621 BA Eindhoven (NL)
(72) Inventor: BRAUERS, Andreas, C/o Philips Int. Pty. & Sds GmbH, 52066 Aachen (DE); WAFFENSCHMIDT, Eberhard, C/o Philips Int. Pty. & Sds GmbH, 52066 Aachen (DE); TE VRUGT, Juergen, C/o Philips Int. Pty & St. GmbH, 52066 Aachen (DE); LAUTER, Josef, C/o Philips Int. Pty. & Stand. GmbH, 52066 Aachen (DE)
(74) Representative: Schouten, Marcus Maria
(86) International application number: PCT/IB2006/051130
(87) International publication number: WO 2006/111889

(56) References cited:
- WO-A-2004/018986
- US-A- 5 964 720
- US-A1- 2003 114 736
- US-A1- 2004 046 668

## Description

The present invention relates to a patient monitoring system, a patient monitoring method and a computer program.

Monitoring of patients during sleep is a standard tool in various situations, mostly in hospital settings. Different parameters are monitored, depending on the diagnosis. Parameters that are needed often are weight, heart rate, breathing rate, and breathing abnormalities, as well as special movement patterns during sleep. It usually requires a significant technical effort and multiple techniques to measure these quantities.

It is known to measure the patient's weight by introducing scales under or in the bedpost. It is further known to gather information on cardiopulmonary performance e.g. by means of pressure-sensitive sheets in a bed. For example, the international patent application WO 2004/045407 A1 describes a device and method for patient monitoring of such parameters as body motion, body position, respiratory rate, and/or heart rate, using a sensor device having at least two piezoelectric sensors provided on a surface, such as a bed, so that a patient can be coupled to the device by simply allowing the patient to lie on the surface.

US2003/0114736 discloses an integrated subject monitoring system facilitating measurement, collection and analysis of data pertaining to frequency and intensity of movement by a recumbent subject.

US2004/0046668 discloses a sensor for use in patient monitoring, wherein the level of patient activity in a chair or bed is tracked to determine whether or not that patient has exhibited sufficient activity to merit eliminating a scheduled assisted relocation to a new position.

It is an object of the present invention to improve the monitoring of patients, particularly for cardiac monitoring and weight monitoring.

This object is achieved according to the invention by a patient monitoring system according to claim 1.

The object of the present invention is also achieved by a patient monitoring method according to claim 7.

The object of the present invention is also achieved by a computer program according to claim 8. The technical effects necessary according to the invention can thus be realized on the basis of the instructions of the computer program in accordance with the invention. Such a computer program may be stored on a carrier such as a CD-ROM or it may be available over the Internet or another computer network. Before being executed, the computer program is loaded into the computer in that it is read from the carrier, for example by means of a CD-ROM player, or from the internet, and is stored in the memory of the computer. The computer includes inter alia a central processor unit (CPU), a bus system, memory means, e. g. RAM or ROM, storage means, e. g. floppy disk or hard disk units and input/output units.

A basic idea of the present invention is to provide a very simple and cheap solution for a combined measurement of weight and cardiopulmonary performance of a patient. In order to achieve this object, it is suggested to use force sensors which are adapted for measuring the forces exerted by a patient on the bed.

In other words, the present invention uses a number of force sensors (e.g. two, three, or four force sensors) to measure and characterize the patient's movements and weight. Special patterns are extracted from these quantities by means of the analyzing module, e.g. heart rate, breathing rate, and special movements such as in restless leg syndrome episodes. Additionally, the presence of the patient in the bed can be deduced. In order to separate these different force signals, it is mandatory to obtain magnitude and direction information of the force vector.

Various kinds of force sensors can be used with the present invention, e.g. strain gauges, piezoelectric devices, capacitive force sensors, etc. Since only a single type of sensors has to be used, a very simple system design can be achieved compared with solutions known from the prior art.

A bed according to the present invention is defined as a surface or any other device to rest on or to sit on, etc., e.g. a conventional bed, a hospital bed, a couch, a conventional chair, a dentist's chair, a wheelchair, an (operating) table, etc.

These and other aspects of the invention will be further elaborated on the basis of the following embodiments which are defined in the dependent claims.

According to a preferred embodiment of the invention, the force sensors are adapted for generating first signals corresponding to a vertical force component and further adapted for generating second signals corresponding to a horizontal force component. In other words, the patient monitoring system is adapted for resolving the measured force into a horizontal component and a vertical component. As a result, the patient's weight can be determined (mainly by using the vertical force component). Furthermore, by separating horizontal and vertical force components, the patient's movements can be determined (mainly by using the horizontal force component). In the case of ballistocardiographic measurements the anisotropic character of the heart movement towards the aorta will deliver a significant horizontal force component while the patient is in a recumbent position. A significant horizontal force component will be provided typically also by a restless leg syndrome movement.

Preferably, the analyzing module is adapted not only for deriving the patient's weight from said signals. Additionally heart rate, and/or breathing rate, and/or breathing abnormalities, and/or special movement patterns during sleep can be determined.

The number of force sensors is preferably associated with a number of bedposts. In such an embodiment no additional wiring and cables are necessary, in particular if also the analyzing module is integrated into the bed or if the data communication between the sensors and the analyzing module and/or between the analyzing module and a display unit or the like is provided in a wireless manner. Alternatively, the set of force sensors may be positioned in a technically equivalent position, e.g. between the mattress and the bed frame.

In another embodiment of the present invention, the force sensors are arranged such that the planes of application of the forces are not parallel to each other, in order to resolve the effective force. In a basic arrangement, the force sensors are simply integrated into the bedposts in a somewhat tilted arrangement. It has been found, however, that the directions of force components be analyzed more easily if the force sensors are arranged such that the planes of application of the forces are orthogonal to each other. In all cases the nature of the sensor arrangement has to be taken into account in deriving the data corresponding to the patient's condition by means of the analyzing module. In other words, the analyzing module, which preferably comprises a computer or equivalent means, e.g. a microprocessor, has to be programmed accordingly.

In yet another embodiment of the invention, the number of force sensors is adapted for measuring and/or shearing forces. In this embodiment the movements of the patient can be determined particularly accurately. Piezoelectric devices are preferably used as force sensors for this purpose.

These and other aspects of the invention will be described in detail hereinafter, by way of example, with reference to the following embodiments and the accompanying drawings; in which
Fig. 1 is a schematic overview showing a patient monitoring system according to the invention,
Fig. 2 is a schematic sectional view of a force sensor arranged in a bedpost as known from the prior art, wherein the force sensor is first unloaded and then loaded,
Fig. 3 is a schematic sectional view of two force sensors arranged in different bedposts according to the present invention, wherein the one force sensor is first unloaded and then loaded,
Fig. 4 is a schematic sectional view of a force sensor arranged in a bedpost according to the present invention, wherein the force sensor is first unloaded and then loaded,
Fig. 5 is a schematic sectional view of another force sensor arranged in a bedpost according to the present invention, wherein the force sensor is first unloaded and then loaded,
Fig. 6 is a schematic sectional view of a force sensor module arranged in a bedpost according to the present invention, wherein the force sensor module comprises three force sensors, the force sensor being first unloaded and then loaded, and
Fig. 7 is a schematic top view of a force sensor module as illustrated in Fig. 6.

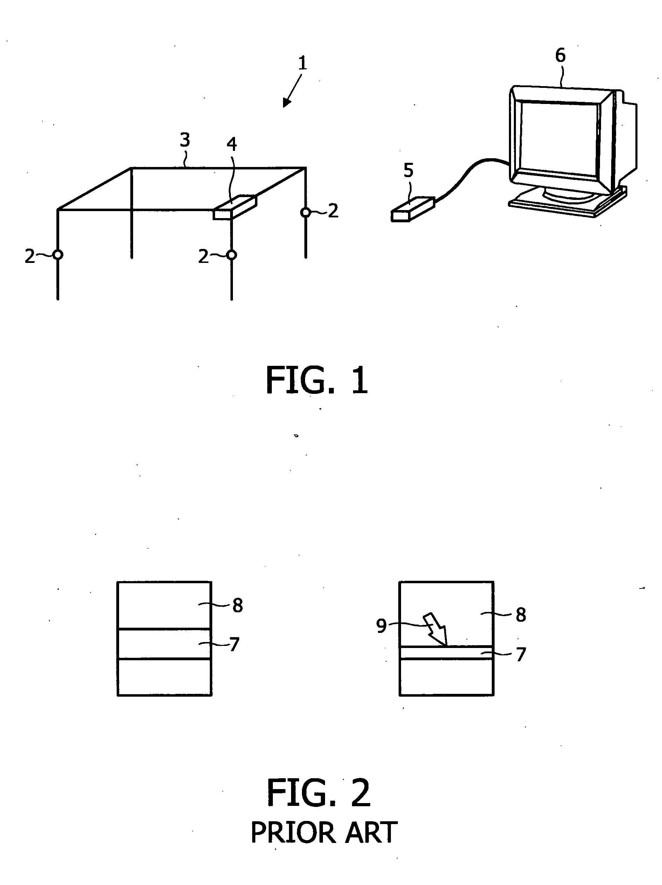

The patient monitoring system 1 comprises three force sensors 2 integrated into the bedposts of a hospital bed 3. Fig. 1 illustrates the frame of such a bed 3 without the mattress. Three piezoelectric sensors are used as the forces sensors 2. With three force sensors 2, a full three-dimensional force model can be reproduced by means of the monitoring system 1. The force sensors 2 are used for measuring the forces exerted on the bed 3. For this purpose the force sensors 2 are positioned such that the planes of application of the forces are orthogonal to each other. In other words, the horizontal and vertical components of the forces are determined. Each force sensor 2 generates signals in dependence on the effective force. Since piezoelectric force sensors 2 are used, the generated signal is a voltage signal corresponding to the deformation of a piezoelectric layer. A first signal is generated if a vertical force component is detected and a second signal is generated if a horizontal force component is detected. The generated signals correspond to the magnitude and direction of the relevant force components.

The patient monitoring system 1 further comprises an analyzing module 4. The analyzing module 4 is mounted to the frame of the bed 3. Signals from the force sensors 2 are transmitted to the analyzing module 4 over a wireless communication path, e.g. based on Bluetooth wireless technology. The analyzing module 4 is adapted for receiving the measured signals and for deriving from these signals data corresponding to the patient's condition. The resulting data are transmitted to an external receiver 5, which is connected to a monitor 6 for displaying the data. Alternative display methods are possible. The measured signals and/or data may be stored within the analyzing unit 4 and/or the external receiving unit 5 and/or some other unit connected to the patient monitoring system 1. For the purpose of deriving patient-related data, the analyzing unit 4 comprises a computer adapted for executing a computer program for deriving these data from the quantity of measured signals. The computer comprises input and output interfaces for data transfer.

The analyzing unit 4 applies the computer program to the signals corresponding to the vertical force component for determining the weight of the patient. Furthermore, the analyzing unit 4 uses the signals corresponding to both the horizontal and the vertical force component to determine the cardiopulmonary performance of the patient, in particular to determine heart rate, breathing rate, and indeed whether the patient is present in the bed 3 at all.

In Fig. 2 a force sensor 7 is illustrated, which is mounted horizontally in a bedpost 8 as known from the prior art (left picture). In this case only the vertical parts of a force 9 acting on the bedpost 8 can be determined (right picture). The effect of the force 9 on the sensor 7 is schematically illustrated as a reduced sensor thickness.

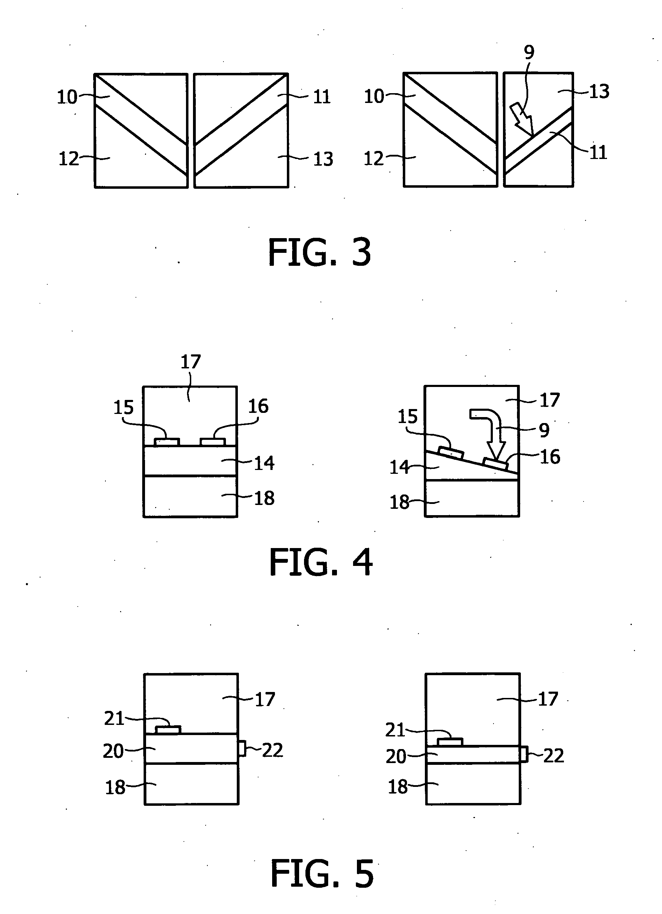

Fig. 3 shows two force sensors 10, 11 according to the present invention. The first force sensor 10 is positioned in a tilted manner in a first bed post 12 and the second force 11 sensor is positioned in the same way in a second bedpost 13, i.e. the force sensors 10, 11 are arranged such that the planes of application of the forces are not parallel to each other, but orthogonal. If no force is exerted, the force sensors 10, 11 are unloaded (left picture). If there is a patient in the bed 3, a force 9 is exerted on the bed frame and on all four bed posts. In the present case there is a patient's movement resulting in a force component towards the second bedpost 13, whereas there is no force component towards the first bedpost 12 (right picture). The result is that the force sensor 11 in the second bedpost 13 determines a force component and generates a relevant signal. The signal comprises the magnitude of the force component. The sensor 11 transmits the signal to the analyzing unit 4 together with a sensor ID (identifier). The analyzing unit 4 knows the geometrical arrangement of all force sensors. Thus the analyzing unit 4 determines from the sensor ID, that the received signal has to be associated with the sensor 11 of the second bedpost 13, and that the received magnitude has to be associated with the effective plane of the force corresponding to this bedpost. The computer program executed in the computer of the analyzing unit 4 processes a complete three-dimensional model of the applied forces. The patient related data (weight, heart rate, etc.) are obtained from the processing of these data.

Fig. 4 shows an embodiment not part of the present invention. This embodiment is based on torque measurement. A piezoelectric layer 14 is provided with two piezoelectric elements (sensors) 15, 16. The piezoelectric layer 14 is located between the upper part 17 and the lower part 18 of a bedpost. The piezoelectric elements 15, 16 are arranged such that the upper part 17 of the bedpost is coupled only to the second piezoelectric element 16, whereas the first piezoelectric element 15 remains uncoupled (left picture). A movement of the patient causes a torque to be applied to the bedposts results in an asymmetrical deformation of the piezoelectric layer 14. As a result, said torque leads to a signal generated by the second piezoelectric element 16, whereas no signal is generated by the first piezoelectric element 15 (right picture). Since said torque also depends on the horizontal component of the applied force, the analyzing module 4 can determine not only the vertical but also the horizontal component of the force from the generated signal.

Fig. 5 shows an embodiment not part of the present invention. This embodiment is based on a measurement of shearing forces. Again a piezoelectric layer 20 is provided, with two piezoelectric elements (sensors) 21, 22, and the piezoelectric layer 20 is located between the upper part 17 and the lower part 18 of a bedpost. The first piezoelectric element 21 is located on top of the piezoelectric layer 20, being coupled to the upper part 17 of the bedpost, whereas the second piezoelectric element 22 is located laterally with respect to the piezoelectric layer 20. In other words, the first piezoelectric element 21 is adapted for measuring the vertical force components only, and the second piezoelectric element 22 is adapted for measuring the horizontal force components only (left picture). If there is a patient movement leading to a purely vertical force, only the first piezoelectric element 21 will generate a relevant signal (right picture), and if there is a patient movement leading to a purely horizontal force, only the second piezoelectric element 22 will generate a relevant signal. However, if there is a horizontal force component as well as a vertical force component, both piezoelectric elements 21, 22 will generate signals, allowing the analyzing module 4 to resolve the effective forces.

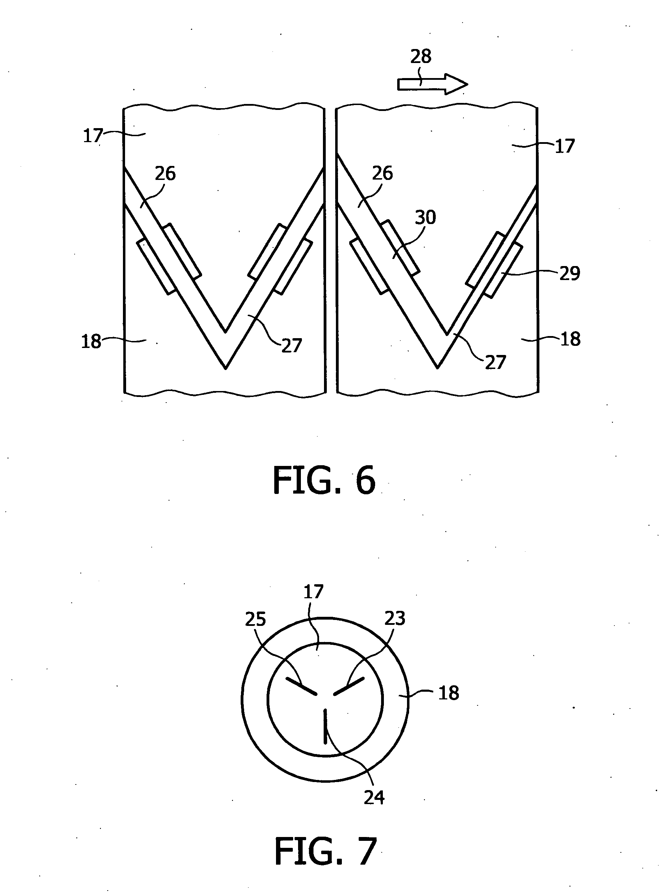

Fig. 6 shows a further embodiment not part of of the present invention. The different planes used for force measurement are realized within a single bedpost here. For this purpose a single sensor module is provided, which is positioned in a single bedpost of the bed 3. In contrast to the arrangement shown in Fig. 1, the other three bedposts do not comprise any force sensors.

The sensor module comprises three force sensors 23, 24, 25 (see Fig. 7). An upper part 17 and a lower part 18 of the bedpost are conical in shape so as to fit into each other. In between there are the three force sensors 23, 24, 25 arranged in a manner that provides measurements along orthogonal planes, in a way as illustrated in Fig. 3. Three piezoelectric layers 26, 27 are located between the upper part 17 and the lower part 18 of the bedpost, and a number of piezoelectric elements (sensors) are provided for each effective plane. If there is no movement, no signal is generated (left picture). If, for example, there is a horizontal force component 28 applied to the upper part 17 of the bedpost, pushing the upper part 17 towards the right, one piezoelectric layer 27 is compressed and a relevant signal is generated by a piezoelectric element 29 of the respective layer 27. At the same time another piezoelectric layer 26 is expanded, leading to the generation of a relevant signal by a piezoelectric element 30 of that layer 26. The analyzing module 4 derives the desired data from these two signals.

Fig. 7 is a plan view of this arrangement. The geometry described just serves as an example. Other geometric arrangements are possible.

Of course, the present invention can be applied not only a patient, but also to a healthy person or to an animal.

It will be evident to those skilled in the art that the invention is not limited to the details of the foregoing illustrative embodiments, and that the present invention may be embodied in other specific forms without departing from the scope of the invention being indicated by the appended claims. Any reference signs in the claims shall not be construed as limiting the claim concerned.

### REFERENCE LIST

- 1: patient monitoring system
- 2: force sensor
- 3: bed
- 4: analyzing module
- 5: receiver
- 6: monitor
- 7: force sensor (prior art)
- 8: bedpost
- 9: acting force
- 10: first force sensor
- 11: second force sensor
- 12: first bedpost
- 13: second bedpost
- 14: piezoelectric layer
- 15: first piezoelectric element
- 16: second piezoelectric element
- 17: upper part of bedpost
- 18: lower part of bedpost
- 19: free
- 20: piezoelectric layer
- 21: first piezoelectric element
- 22: second piezoelectric element
- 23: force sensor
- 24: force sensor
- 25: force sensor
- 26: piezoelectric layer
- 27: piezoelectric layer
- 28: force component
- 29: piezoelectric element
- 30: piezoelectric element

## Claims

1. A patient monitoring system (1), the system comprising a number of force sensors (2) associated with a patient's bed (3), said force sensors (2) being adapted for generating first signals corresponding to a first force component and second signals corresponding to a second force component, the second force component not running parallel to the first force component, which system further comprises an analyzing module (4) adapted for deriving from said signals data indicative of the patient's condition, wherein the patient's bed comprises a first bed post (12) comprising a first force sensor (10) and a second bed post (13) comprising a second force sensor (11), **characterized in that** said first and second force sensor having a plane of application of force being tilted relative to a plane defined by the patient's bed, the planes of application of forces of the first and second force sensor being orthogonal to each other.

2. The patient monitoring system (1) as claimed in claim 1, wherein the analyzing module (4) is adapted for deriving the patient's weight.

3. The patient monitoring system (1) as claimed in claim 1, wherein the analyzing module (4) is adapted for deriving the patient's movements.

4. The patient monitoring system (1) as claimed in claim 1, wherein the analyzing module (4) is adapted for deriving from said signals the patient's heart rate, and/or breathing rate, and/or breathing abnormalities, and/or special movement patterns.

5. The patient monitoring system (1) as claimed in claim 1, wherein the number of force sensors (2) is associated with a number of bedposts.

6. The patient monitoring system (1) as claimed in claim 1, wherein at least two force sensors (23, 24, 25) are arranged such that they form a combined sensor module to be positioned in a single bedpost.

7. A patient monitoring method, the method comprising the steps of:
- assigning a number of force sensors (2) to a patient's bed (3), the force sensors (2) being adapted for generating first signals corresponding to a first force component and second signals corresponding to a second force component, the second force component not running parallel to the first force component, the patient's bed comprising a first bed post (12) comprising a first force sensor (10), a second bed post (13) comprising a second force sensor (11), said first and second force sensor having a plane of application of force being tilted relative to a plane defined by the patient's bed, the planes of application of forces of the first and second force sensor being orthogonal to each other, and
- deriving from said signals data indicative of the patient's condition.

8. A computer program comprising computer instructions for deriving data from a number of signals when said computer program is executed on a computer (4), said data corresponding to a patient's condition, said signals corresponding to a first force component and a second force component, the second force component not running parallel to the first force component, said force components being generated by a number of force sensors (2) associated with a patient's bed (3), the patient's bed comprising a first bed post (12) comprising a first force sensor (10), a second bed post (13) comprising a second force sensor (11), said first and second force sensor having a plane of application of force being tilted relative to a plane defined by the patient's bed, the planes of application of forces of the first and second force sensor being orthogonal to each other.

## Patentansprüche

1. Patientenüberwachungssystem (1), das eine Anzahl von zu einem Patientenbett (3) gehörenden Kraftsensoren (2) umfasst, wobei die genannten Kraftsensoren (2) vorgesehen sind, um erste Signale, die einer ersten Kraftkomponente entsprechen, und zweite Signale, die einer zweiten Kraftkomponente entsprechen, zu erzeugen, wobei die zweite Kraftkomponente nicht parallel zu der ersten Kraftkomponente verläuft, wobei das System weiterhin ein Analysemodul (4) umfasst, das vorgesehen ist, um von den genannten Signalen Daten abzuleiten, die auf den Zustand des Patienten hinweisen, wobei das Patientenbett einen ersten Bettpfosten (12) mit einem ersten Kraftsensor (10) und einen zweiten Bettpfosten (13) mit einem zweiten Kraftsensor (11) umfasst, **dadurch gekennzeichnet, dass** der genannte erste und zweite Kraftsensor eine Ebene der Kraftausübung haben, die in Bezug auf eine durch das Patientenbett definierte Ebene geneigt ist, wobei die Ebenen der Kraftausübung des ersten und des zweiten Kraftsensors orthogonal zueinander sind.

2. Patientenüberwachungssystem (1) nach Anspruch 1, wobei das Analysemodul (4) vorgesehen ist, um das Gewicht des Patienten abzuleiten.

3. Patientenüberwachungssystem (1) nach Anspruch 1, wobei das Analysemodul (4) vorgesehen ist, um die Bewegungen des Patienten abzuleiten.

4. Patientenüberwachungssystem (1) nach Anspruch 1, wobei das Analysemodul (4) vorgesehen ist, um von den genannten Signalen die Herzfrequenz und/oder Atemfrequenz und/oder Atmungsanomalien und/oder spezielle Bewegungsmuster des Patienten abzuleiten.

5. Patientenüberwachungssystem (1) nach Anspruch 1, wobei die Anzahl der Kraftsensoren (2) zu einer Anzahl von Bettpfosten gehört.

6. Patientenüberwachungssystem (1) nach Anspruch 1, wobei mindestens zwei Kraftsensoren (23, 24, 25) derart vorgesehen sind, dass sie ein kombiniertes Sensormodul bilden, das in einem einzigen Bettpfosten anzuordnen ist.

7. Patientenüberwachungsverfahren, wobei das Verfahren die folgenden Schritte umfasst:
- Zuweisen einer Anzahl von Kraftsensoren (2) zu einem Patientenbett (3), wobei die Kraftsensoren (2) vorgesehen sind, um erste Signale, die einer ersten Kraftkomponente entsprechen, und zweite Signale, die einer zweiten Kraftkomponente entsprechen, zu erzeugen, wobei die zweite Kraftkomponente nicht parallel zu der ersten Kraftkomponente verläuft, wobei das Patientenbett einen ersten Bettpfosten (12) mit einem ersten Kraftsensor (10) und einen zweiten Bettpfosten (13) mit einem zweiten Kraftsensor (11) umfasst, wobei der genannte erste und zweite Kraftsensor eine Ebene der Kraftausübung haben, die in Bezug auf eine durch das Patientenbett definierte Ebene geneigt ist, wobei die Ebenen der Kraftausübung des ersten und des zweiten Kraftsensors orthogonal zueinander sind, und
- Ableiten von Daten, die auf den Zustand des Patienten hinweisen, von den genannten Signalen.

8. Computerprogramm mit Computeranweisungen zum Ableiten von Daten von einer Anzahl von Signalen, wenn das genannte Computerprogramm auf einem Computer (4) ausgeführt wird, wobei die genannten Daten einem Patientenzustand entsprechen, wobei die genannten Signale einer ersten Kraftkomponente und einer zweiten Kraftkomponente entsprechen, wobei die zweite Kraftkomponente nicht parallel zu der ersten Kraftkomponente verläuft, wobei die genannten Kraftkomponenten durch eine Anzahl von zu einem Patientenbett (3) gehörenden Kraftsensoren (2) erzeugt werden, wobei das Patientenbett einen ersten Bettpfosten (12) mit einem ersten Kraftsensor (10) und einen zweiten Bettpfosten (13) mit einem zweiten Kraftsensor (11) umfasst, wobei der genannte erste und der zweite Kraftsensor eine Ebene der Kraftausübung haben, die in Bezug auf eine durch das Patientenbett definierte Ebene geneigt ist, wobei die Ebenen der Kraftausübung des ersten und des zweiten Kraftsensors orthogonal zueinander sind.

## Revendications

1. Système de surveillance de patient (1), le système comprenant un nombre de capteurs de force (2) associés à un lit du patient (3), lesdits capteurs de force (2) étant adaptés pour générer des premiers signaux correspondant à une première composante de force et des seconds signaux correspondant à un seconde composante de force, la seconde composante de force n'étant pas parallèle à la première composante de force, lequel système comprend en outre un module d'analyse (4) adapté pour dériver, à partir desdits signaux, des données indicatives de la condition d'un patient, dans lequel le lit du patient comprend un premier montant de lit (12) comprenant un premier capteur de force (10), et un second montant de lit (13) comprenant un second capteur de force (11), **caractérisé en ce que** lesdits premier et second capteurs de force possèdent un plan d'application de force incliné par rapport à un plan défini par le lit du patient, les plans d'application de forces des premier et second capteurs de force étant orthogonaux l'un à l'autre.

2. Système de surveillance de patient (1) selon la revendication 1, dans lequel le module d'analyse (4) est adapté pour dériver le poids du patient.

3. Système de surveillance de patient (1) selon la revendication 1, dans lequel le module d'analyse (4) est adapté pour dériver les mouvements du patient.

4. Système de surveillance de patient (1) selon la revendication 1, dans lequel le module d'analyse (4) est adapté pour dériver, à partir desdits signaux, le rythme cardiaque du patient, et/ou le rythme respiratoire, et/ou des anormalités respiratoires, et/ou des modes de comportement de mouvement spéciaux.

5. Système de surveillance de patient (1) selon la revendication 1, dans lequel le nombre de capteurs de force (2) est associé à un nombre de montants de lit.

6. Système de surveillance de patient (1) selon la revendication 1, dans lequel au moins deux capteurs de force (23, 24, 25) sont agencés de sorte qu'ils forment un module de capteur combiné destiné à être positionné dans un seul montant de lit.

7. Procédé de surveillance de patient, le procédé comprenant les étapes consistant à :
- attribuer un nombre de capteurs de force (2) à un lit du patient (3), les capteurs de force (2) étant adaptés pour générer des premiers signaux correspondant à une première composante de force et des seconds signaux correspondant à une seconde composante de force, la seconde composante de force n'étant pas parallèle à la première composante de force, le lit du patient comprenant un premier montant de lit (12) comprenant un premier capteur de force (10), un second montant de lit (13) comprenant un second capteur de force (11), lesdits premier et second capteurs de force possédant un plan d'application de force incliné par rapport à un plan défini par le lit du patient, les plans d'application de forces des premier et second capteurs de force étant orthogonaux l'un à l'autre, et
- dériver, à partir desdits signaux, des données indicatives de la condition d'un patient.

8. Programme d'ordinateur comprenant des instructions informatiques pour dériver des données à partir d'un nombre de signaux lorsque ledit programme d'ordinateur est exécuté sur un ordinateur (4), lesdites données correspondant à la condition d'un patient, lesdits signaux correspondant à une première composante de force et une seconde composante de force, la seconde composante de force n'étant pas parallèle à la première composante de force, lesdites composantes de force étant générées par un nombre de capteurs de force (2) associés à un lit du patient (3), le lit du patient comprenant un premier montant de lit (12) comprenant un premier capteur de force (10), un second montant de lit (13) comprenant un second capteur de force (11), lesdits premier et second capteurs de force possédant un plan d'application de force incliné par rapport à un plan défini par le lit du patient, les plans d'application de forces des premier et second capteurs de force étant orthogonaux l'un à l'autre.
